# EUROPEAN PATENT APPLICATION

(11) **EP 1 374 693 A2**
(43) Date of publication of application: **02.01.2004**
(21) Application number: 03012834.2
(22) Date of filing: 05.06.2003
(51) Int. Cl.: A23K 1/00, A61K 31/198, A61P 15/14

(54) **Method for preventing decrease of breast milk amount in mammals**

(30) Priority: 06.06.2002 JP 2002165492
(71) Applicant: Ajinomoto Co., Inc., Tokyo (JP)
(72) Inventor: Shinzato, Izuru, Kawasaki-shi, Kanagawa 210-8681 (JP); Kobayashi, Hisamine, Kawasaki-shi, Kanagawa 210-8681 (JP); Shibahara, Susumu, Kawasaki-shi, Kanagawa 210-8681 (JP); Nishimura, Naoki, Kawasaki-shi, Kanagawa 210-8681 (JP)
(74) Representative: Strehl Schübel-Hopf & Partner

(57) **Abstract**

A method for preventing decrease of a breast milk amount in a mammal, which comprises administering glutamine to the mammal.

## Description

### BACKGROUND OF THE INVENTION

### 1. Field of the Invention

The present invention relates to a method for preventing decrease of a breast milk amount in mammals.

### 2. Brief Description of the Background Art

Mammals secrete breast milk after parturition. When mammals are under poor physical conditions, suffer from diseases or are under poor nutritional conditions, their milk secretion is decreased and subsequently growth or nutritional status of their babies is deteriorated. In the case of economic animals, such as a cow or a goat, of which milk and processed products thereof are edible for human beings, the decrease of the milk amount leads to a great economic loss and serious problems take place for dairy farmers. In particular, when high-producing daily cows suffer from metabolic diseases during periparturient period, e.g., retained placenta, milk fever, ketosis, displaced abomasum and mastitis, or when they are under sub-clinical disease conditions even if they are not actually showing symptoms of these diseases, great problems are caused because their milk amounts are decreased and it takes long to be recovered therefrom. Methods for preventing decrease of a breast milk amount in economic animals include a method such as feeding a large amount of ingredients having a high energy value such as sugar and starch to mammals to thereby increase the intake of energy in the mammals, and supplying a feed to mammals containing a lot of protein such as fish meal and blood meal. However, these methods are all inexpensive but are not so effective. In the case of ketosis caused in daily cows under poor nutritional conditions, intravenous injection of saccharides such as glucose is known as an ordinary treatment method. However, the effect is temporarily, and the amount of milk which has been once decreased cannot be completely restored.

### SUMMARY OF THE INVENTION

An objective of the present invention is to provide a novel method which is inexpensive and safe for preventing decrease of an breast milk amount in mammals.

Another objective of the present invention is to provide a method for preventing decrease of a milk amount in high producing daily cows when they suffer from metabolic diseases during periparturient period, particularly when they suffer from ketosis or from sub-clinical ketosis which are close to ketosis even if they have no symptoms.

These and other objects of the present invention have been accomplished by a method for preventing decrease of a breast milk amount in a mammal, which comprises administering glutamine to the mammal.

### BRIEF DESCRIPTION OF THE DRAWING

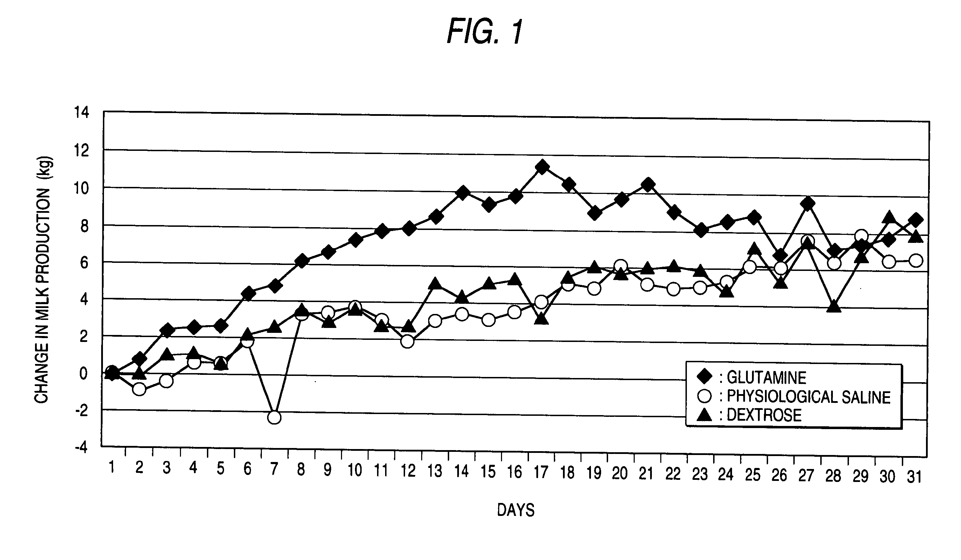

Fig. 1 shows the change of daily milk production after administration in Example and Comparative Example. The X-axis indicates days after administration, and the γ-axis indicates the change of the milk amount based on that on the first day of the test (day of administration).

### DETAILED DESCRIPTION OF THE INVENTION

The inventors of the present invention made extensive studies to solve the above problems. As a result, they found that decrease of a breast milk amount in mammals can be prevented by injection or oral administration of glutamine. The present invention has thus been completed.

That is, the present invention provides a novel method for preventing decrease of a breast milk amount in mammals by administration of glutamine. This method is particularly effective as a method for preventing decrease of a breast milk amount when mammals are under poor physical conditions or nutritional conditions.

Furthermore, the present invention provides a method preventing decrease of a milk amount in high producing daily cows when they suffer from metabolic diseases during periparturient period, particularly when they suffer from ketosis or from sub-clinical ketosis which are close to ketosis even if they have no symptoms, by administration of glutamine.

The method of the present invention can be effectively applied not only to mammals of which milk per se and processed dairy products such as cheese and yogurt are edible, e.g., a cow, a goat, a sheep and a water buffalo, but also to animals for meat production such as a pig and a horse because inhibition of decrease of the breast milk amount leads to improvement of growth in babies which consume the breast milk. Furthermore, the method of the present invention can be applied to all mammals including a human in which a breast milk amount is decreased due to poor nutritional conditions or physical conditions.

The administration of glutamine can quickly exert its effect by injecting an(aqueous solution of glutamine. Also, glutamine may be orally administered. The dose of glutamine is from 100 mg/kg to 5 g/kg, preferably from 500 mg/kg to 1 g/kg, per day as calculated in terms of glutamine. With regard to the frequency of administration, since even single administration can prevent decrease of a breast milk amount for several days to about two weeks, glutamine may be administered as the occasion demands during the lactation period. In practice, about one shot per week is effective. If glutamine is administered every day, no adverse effects are caused and similar effects can be obtained. The timing of administration is not particularly limited. -In practice, it is more effective to administer glutamine to mother animals during the period from one month before parturition to the lactation period, when they tend to be under low nutritional conditions.

In the present invention, glutamine is preferably L-glutamine. Glutamine may by used in the form of a free amino acid or in the form of a compound or a mixture of a physiologically available salt. Also, glutamine may be used in the form of a peptide containing glutamine such as alanyl-glutamine and glycyl-glutamine. Furthermore, a protein hydrolysate containing glutamine, such as a hydrolysate of a wheat protein which has a high glutamine content, may be used. Herein, the peptide and the protein hydrolysate used. in the present invention, including amino acid numbers, molecular weights, properties and the like, are not particularly limited so long as they include glutamine, and preferably, they can release glutamine in the body after the administration. Also, other amino acids and the like constituting the peptide and the protein hydrolysate are not particularly limited. The protein hydrolysate is preferably has a molecular weight of 2,000 or less. Moreover, various physiologically available glutamine derivatives such as acetylglutamine may be used.

Glutamine is a nutritionally nonessential amino acid. Unlike a method in which only essential amino acids which are marginal and limited in feeds, such as methionine and lysine, are added to a feed and the feed is orally administered, the method of the present invention has such a great economical advantage that the administration time is short.

When glutamine is used in an injection, the injection may contain inorganic salts, saccharides and the like for the purpose of adjusting its pH or osmotic pressure. Solvents used for the injection include distilled water for injection, a physiological saline, vegetable oil, propylene glycol, polyethylene glycol, alcohols such as ethanol, and mixtures thereof. The injection may be preferably carried out intravenously.

When administered orally, glutamine may be added to ordinary feeds, aqueous solutions, drinks such as juice, soft drinks and milk, or solid foods such as sweets. Glutamine may be used in the form of tablets, capsules, granules, powder and the like. If necessary, glutamine may be used together with additives, for example, binders such as tragacanth gum, gum arabic, corn starch and gelatin; diluting agents such as microcrystalline cellulose; swelling agents such as corn starch, gelatinized starch and alginic acid; lubricants such as magnesium stearate; sweeteners such as sugar, lactose and aspartame; and flavors such as peppermint, *Gaultheria adenothrix* oil and cherry.

When administered orally to ruminants, degradation in the rumen must be avoided, so glutamine may be used in the form of rumen-protected preparations obtained by a commonly known method such as coating granule.

The present invention is described below based on examples; however, the present invention is not limited thereto.

### Example

Ten lactating Holstein cows in the immediate post-partum period of which milk production was 10-15% decreased in comparison with that of one week before the beginning of test due to sub-clinical ketosis were used. All cows were supplied with a complete feed for lactating cows composed of corn silage and concentrate containing grains, oil meals, animal proteins and vitamins/minerals. On the first day in the test, a solution containing 29 g of L-glutamine dissolved in 1 liter of distilled water for injection was injected into the cows through the cervical vein. The cows were then milked twice a day for one month, and the milk amount was measured.

### Comparative Example

Ten lactating Holstein cows in sub-clinical ketosis were selected as negative controls according to the same criterion as in Example. On the first day in the test, 1 liter of saline was injected through the cervical vein as the negative control. On the other hand, nine lactating Holstein cows selected according to the same criterion as described above were used. On the first day in the test, 1 liter of a 25% dextrose solution was injected through the cervical vein as the positive control. Both the negative and positive controls were then supplied with the same complete feed as used in Example. The cows were then milked twice a day for one month, and the milk amount was measured.

Fig. 1 shows the change of the milk amount after administration. The X-axis indicates days after administration, and the Y-axis indicates the increment of the milk amount from that on the first day of the test. In the saline-administered group, the milk amount was slightly decreased on the 2nd and 3rd days after administration. Although the milk amount was gradually increased on and after the 8th day, the increase was about 8 kg per day at maximum. In the dextrose-administered group, the milk amount was on substantially the same level up to the 5th day after administration but gradually increased on and after the 6th day, and the increase was about 9 kg per day at maximum. On the other hand, in the glutamine-administered group, the milk amount was increased immediately after administration, and this tendency lasted even one month after administration. The increase of the milk amount was about 12 kg per day at maximum. On the average, the saline-administered group and the dextrose-administered group showed milk amount increase at 3.9 kg arid 4.5 kg, respectively, per day from that on the first day of the test, while the glutamine-administered group showed increase as much as 7.6 kg per day. Thus, the present invention can provide a novel method which is inexpensive and safe for preventing decrease of milk amount in mammals.

This application is based on Japanese applications No. 2002-165492 filed.on June 6, 2002, the entire contents of which are incorporated hereinto by reference.

While the invention has been described in detail and with reference to specific embodiments thereof, it will be apparent to one skill in the art that various changes and modifications can be made therein without departing from the spirit and scope thereof. All references cited herein are incorporated, by reference, in their entirety.

## Claims

1. A method for preventing decrease of a breast milk amount in a mammal, which comprises administering glutamine to the mammal.

2. The method according to claim 1, wherein the glutamine is administered at a dose of from 100 mg/kg to 5 g/kg per day as calculated in terms of glutamine.

3. The method according to claim 1 or 2, wherein the decrease of a breast milk amount is caused by poor physical conditions or poor nutritional conditions of the mammal.

4. The method according to any one of claims 1 to 3, wherein the decrease of a breast milk amount is caused by metabolic diseases at a periparturient period of the mammal.

5. The method according to any one of claims 1 to 4, wherein the decrease of a breast milk amount is caused by ketosis or sub-clinical ketosis.

6. The method according to any one of claims 1 to 5, wherein the mammal is selected from the group consisting of a cow, a goat, a sheep, a water buffalo, a swine and a horse.

7. The method according to any one of claims 1 to 5, wherein the mammal is a human.

8. The method according to any one of claims 1 to 7, wherein the glutamine is L-glutamine.

9. The method according to any one of claims 1 to 8, wherein the glutamine is administered by injection.

10. The method according to any one of claims 1 to 8, wherein the glutamine is orally administered.
